# EUROPEAN PATENT APPLICATION

(11) **EP 3 111 939 A1**
(43) Date of publication of application: **04.01.2017**
(21) Application number: 14876219.8
(22) Date of filing: 25.12.2014
(51) Int. Cl.: A61K 31/59, A61K 9/00, A61P 13/10, A61P 13/02

(54) **VITAMIN D AND ANTIBACTERIAL USES OF COMPOSITION THEREOF**

(30) Priority: 02.01.2014 CN 201410001937
(71) Applicant: Zensun (Shanghai) Science & Technology, Co., Ltd., Zhangjiang Hi-Tech Park Pudong Shanghai 201203 (CN)
(72) Inventor: ZHOU, Mingdong, Bexley, New South Wales 2207 (AU)
(74) Representative: Jones Day
(86) International application number: PCT/CN2014/094893
(87) International publication number: WO 2015/101208

(57) **Abstract**

Uses of a vitamin D compound in the preparation of a medicament for preventing or treating cystitis, urethritis and/or urinary tract infection, as well as a pharmaceutical composition, a pharmaceutical preparation and a kit that comprise the vitamin D compound, wherein the vitamin D compound is selected from vitamin D2, vitamin D3, and an active form or analog of the vitamin D3, and wherein the active form of vitamin D₃ is 25-hydroxyvitamin D₃ or 1,25-dihydroxyvitamin D₃.

## Description

### FIELD OF THE INVENTION

The present invention relates to the use of a Vitamin D compound in the preparation of a medicament for preventing or treating cystitis, urethritis and/or urinary tract infection. Particularly, the present invention relates to pharmaceutical compositions and pharmaceutical preparations for preventing or treating cystitis, urethritis and/or urinary tract infection.

### BACKGROUND OF THE INVENTION

Urinary tract infection (UTI) refers to a kind of inflammation caused by pathogens growing and proliferating in urinary track and affecting mucosa or organization of urinary tract, and commonly seen as bacterial UTI. UTI is a common clinical disease. According to the hospital outpatient medical investigation and the outpatient medical investigation in the USA, there are approximate 7 million UTI outpatients and 1 million emergency cases every year, among which 100 thousands patients require hospitalization, especially female patients are in the majority. It is reported that the recurrence rate of UTI is high, and about 25% to 30% of female patients have a recurrent infection. Moreover, some researches show that up to 44.1% female patients have a recurrence within a year. The high recurrence rate of UTI results in significant economic losses. It is estimated by scholars that the costs of treating UTI are up to 1.6 billions of dollors every year in the USA.

Urethritis is a common disease referring to the inflammation of the urinary mucosa, which occured more often in women. Urethritis can be classified as acute urethritis, chronic urethritis, nonspecific urethritis and gonococcus sex urethritis in clinical practice. Most urethritis is caused by pathogenic bacteria retrograding into urethral canal. Urethritis would occur when bacteria enter into bladder or kidney and reproduce in urine.

The most common UTI is urocystitis, i.e., the inflammation occurs in bladder. Bacterial urocystitis is the most common one. The causes of bacterial urocystitis is that bacteria on the body surface invade into urethral orifice, and proliferate in bladder, with the symptoms of pollakiuria, urgent urination, pain or burning sensation during urination, and hematuresis. The process of the disease outbreak is mostly rapid, and it will cause severe discomfort within half a day, even spread to the kidneys to develop into acute pyelonephritis in a few days. E. coli is the most common bacteria that cause cystitis. It is easier for bacteria to breed in the bladder, particularly under the condition of drinking insufficient water or holding back urine.

Under normal circumstances, pathogenic bacteria are not easy to stay and breed in the urinary tract, so they will not cause infection. This is due to the physiological defense function of urinary and reproductive system, the most important of which is the secretion of antimicrobial peptides. Once the pathogens invade, antimicrobial peptides have bactericidal effect, and this reaction is quite fast, so that bacteria can be cleared before breeding in the body. But once this anti-infective defense weakens, pathogenic bacteria will invade into body and induce infection.

Antimicrobial peptides, usually comprising 20 to 60 amino acids, are generally positively charged and amphiphilic. Amphipathicity makes them achieve higher concentration in both aqueous environment and hydrophobic environment (such as membrane). Antimicrobial peptides can be quickly adsorbed by the membrane of incursive microorganisms and kill them. Nowadays the majority human antimicrobial peptides studies are focus on defensin and cathelicidin (also known as LL-37). Normally low level of cathelicidin can be detected in urinary tract. Once microbial invading, the expression of mRNA encoding cathelicidin increase rapidly. Chromek et al. infected cathelicidin gene knockout mice and wild type mice with E. coli separately. The result showed that bacteria count of the gene knockout mice was significantly higher than that of the wild type mice in urethra.

Vitamin D plays an important role in human nutrition. It is a kind of fat-soluble vitamin. Vitamin D₂ (Ergocalciferol) and vitamin D₃ (cholecalciferol) are the two main forms existing in body. People mainly rely on food intake and synthesis from body skin for obtaining vitamin D. 7 -dehydrocholesterol in the skin can synthesize vitamin D precursor under the UV irradiation of 290 ∼ 320mm wavelength. The two forms of vitamin D cannot be long-term cycle in the blood and will be absorbed by adipose tissue or liver for store or activation. The activated vitamin D binds to its receptor VDR and enters into the nucleus to regulate various genes expression. VDR is widespread in various human cells, so vitamin D has a wide range of biological effects, such as functions of promoting the absorption of calcium and phosphorus which was known in the past and functions of promoting cell differentiation, inhibiting cell proliferation, promoting the secretion of antimicrobial peptides, enhancing innate immunity, specific immune suppression and so on, which have been found in recent years.

Direct solar radiation to the skin is the main way to produce natural vitamin D for the body, but because of the change of modern social life habits, the modern people stay indoors longer and use more sunscreen products, so that people have fewer opportunities to receive enough sunlight. Moreover, food sources of vitamin D are very limited (such as cod liver oil, liver of some aquatic mammals and fat et al.), so most people cannot supplement vitamin D from the regular diet, resulting in a general worldwide trend of the lack of vitamin D currently. A number of large-scale epidemiological survey abroad (including the United States, Canada, Britain, France, Germany, Spain, Russia, New Zealand and Israel et al.) showed that about 50% to 70% of people in the state of vitamin D deficiency among older children, youth and middle-aged adults. In China, the situation of vitamin D deficiency is more severe, especially when the diet structure of Chinese is lack of food rich in vitamin D, and the opportunity of exposure to sunlight and outdoor in modern life is also greatly reduced, so the probability of vitamin D deficiency among Chinese inhabitants is higher than the Westerners. According to the study of Chinese Academy of Sciences, the phenomenon of Vitamin D deficiency is even more serious in elder population. The survey results showed that blood level of vitamin D of the elderly was overall low, where vitamin D deficiency accounted for 93.6%, while vitamin D sufficiency accounted for only 6.4%

Vitamin D deficiency or insufficiency has extremely negative impact on the body's immunity and reduces resistance to recurrent urinary tract infection. Though no direct evidences show that vitamin D deficiency can lead to decreased immunity as confirmed by epidemiological data, some epidemiological studies have shown that the decrease of 25 (OH) D serum concentration in vivo could increase infection rate of upper respiratory tract. Other epidemiological studies have shown that vitamin D deficiency in mother and infant is associated with high morbidity of acute lower respiratory tract infections in infants. The studies of Gibney et al. have shown that vitamin D deficiency associated with the incidence of tuberculosis. These studies reveal indirectly that vitamin D deficiency or insufficiency causes some negative impact on immune system. In addition, pregnant women and elderly women are of the population at high risk of vitamin D deficiency or insufficiency. The Studies have shown that 2% to 10% of pregnant women and up to 20% of women over 80 years have asymptomatic bacteriuria, whose incidence probability are far more than ordinary people, suggesting that there is some intrinsic link between them.

Urinary tract infections can be classified into urethritis, cystitis, pyelonephritis according to the infection site of pathogen. Severe pyelonephritis leads to renal damage and even renal failure. Gram-negative bacillus sepsis is usually caused by urinary tract infection. A few sepsis patients can occur obvious shock, accompanied by clinical manifestations such as the heart, brain and kidney ischemia.

Now cystitis, urethritis and / or urinary tract infections mainly treated by antibiotic therapy. Commonly used antibiotics comprise TMP-SMX, Nitrofurantoin, ciprofloxacin, levofloxacin or chemical analogue thereof, some penicillin such as amoxicillin. Although antibiotics can reduce cystitis, urethritis and / or urinary tract infection to some extent, the cost of antibiotics widely used is equally high, which results in increased drug-resistant bacteria and decreased number of normal bacteria in patients. One study showed that the resistance to ciprofloxacin, amoxicillin, clavulanic acid, TMP-SMX of E. coli ,isolated from patients with cystitis, urethritis and/or urinary tract infection, had been significantly increased during the 1998-2003 five-year period. Abuse of antibiotics will lead to the increase of drug-resistant bacteria. Because of the bacterial resistance, more antibiotics or stronger antibiotics are used clinically, resulting in economic waste; and nonspecific bactericidal action also leads to a reduction of beneficial bacteria in vivo, resulting in unnecessary harm to the human body. Antibiotics can quickly suppress the proliferation of bacteria, but cannot increase the body's innate immunity. When pathogens invade again, innate immunity in vivo is still not sufficient to kill pathogens, so infections are easy to recur. Antibiotic therapy alone cannot reduce the high recurrence rate of cystitis, urethritis and/or urinary tract infection as mentioned above, which could easily lead to significant economic losses and waste of medical resources. So now we need a new effective prevention and treatment method which can be used alone or in a combination of different antibiotics for reducing the high recurrence rate of cystitis, urethritis and/or urinary tract infections.

A major cause of cystitis, urethritis and/or urinary tract infection is the decline of inherent natural immune system. One approach to reduce recurrence rate of cystitis, urethritis and/or urinary tract infection is to enhance the innate immunity. The active vitamin D combined with VDR (vitamin D receptor) enters into the nucleus, acts on a variety of target genes to enhance people's innate immunity, regulates the expression of antimicrobial peptides, inhibits bacteria adhesion, kills or inhibits pathogens, reduces the recurrence rate. The theoretical basis and experimental results make it possible to develop new drugs for prevention and treatment of cystitis, urethritis and/or urinary tract infections. The present application provides pharmaceutical preparations for effectively reducing recurrent infections of cystitis, urethritis and/or urinary tract. The decrease of recurrence of cystitis, urethritis and/or urinary tract infections can improve the quality of life of patients and reduce antibiotic applications to ease the current situation of clinical antibiotics abuse. The use of Vitamin D compound in the preparation for the prevention or treatment of cystitis, urethritis and/or urinary tract infection medicament will greatly change the situation of prevention and treatment of cystitis, urethritis and/or urinary tract infection.

### DESCRIPTION OF THE INVENTION

### A. Summary of the Invention

The present invention relates to the use of a Vitamin D compound in the preparation of a medicament for preventing or treating cystitis, urethritis and / or urinary tract infections. The present invention relates to the use of a Vitamin D compound in the preparation of a medicament for preventing or treating cystitis. The present invention relates to the use of a Vitamin D compound in the preparation of a medicament for preventing or treating urethritis. The present invention relates to the use of a Vitamin D compound in the preparation of a medicament for preventing or treating urinary tract infections, wherein urinary tract infections comprises upper urinary tract infection and lower urinary tract infections. According to the infection site of pathogens, urinary tract infections can be classified into urethritis, cystitis and pyelonephritis. In a preferred embodiment, the vitamin D compound is a vitamin D receptor ligand (VDR ligand). In a more preferred embodiment, the vitamin D compound is a vitamin D receptor agonist. Preferably, the vitamin D compound is selected from vitamin D₂, vitamin D₃, as well as an isomer, activated form or analog thereof. Preferably, the vitamin D compound is selected from vitamin D₂, D₃ and their analogs. In certain embodiments, the vitamin D compound is an activated form of vitamin D, e.g., calcifediol (25-OH D₃) or calcitriol (1,25- (OH)₂ D₃).

In one aspect, the present invention relates to a pharmaceutical composition for preventing or treating cystitis, urethritis and/or urinary tract infections. The composition comprises a vitamin D compound and a second drug for the prevention or treatment of cystitis, urethritis, and/or urinary tract infections. The present invention relates to a pharmaceutical composition for preventing or treating cystitis. The composition comprises a vitamin D compound and a second drug for the prevention or treatment of cystitis. The present invention relates to a pharmaceutical composition for preventing or treating urethritis. The composition comprises a vitamin D compound and a second drug for the prevention or treatment of cystitis. The present invention relate to a pharmaceutical composition for preventing or treating urinary tract infections. The composition comprises a vitamin D compound and a second drug for the prevention or treatment of urinary tract infections. In a preferred embodiment, the vitamin D compound is a vitamin D receptor ligand (VDR ligand). In a more preferred embodiment, the vitamin D compound is a vitamin D receptor agonist. Preferably, the vitamin D compound is selected from vitamin D₂, vitamin D₃, as well as an isomer, activated form or analog thereof. Preferably, a vitamin D compound is selected from vitamin D₂, D₃ and their analogs. In certain embodiments, the vitamin D compound is an activated form of vitamin D, e.g., calcifediol (25-OH D₃) or calcitriol (1,25- (OH)₂ D₃). In a preferred embodiment, the second drug for the prevention or treatment of cystitis, urethritis and/or a urinary tract infection is an antibiotic such as TMP-SMX, nitrofurantoin, ciprofloxacin, levofloxacin or chemical analogue thereof, penicillin, and amoxicillin. In certain embodiments, the pharmaceutical compositions of the present invention are suitable for topical or oral administration to a subject. In other embodiments, as described hereinafter, the pharmaceutical compositions of the invention may be specially formulated as a solid or liquid form for administration , such as oral administration, parenteral administration, topical administration, etc.

In another aspect, the present invention provides a pharmaceutical preparation for preventing or treating cystitis, urethritis and/or urinary tract infections, said pharmaceutical preparation comprises a vitamin D compound and pharmaceutically acceptable carriers. The present invention provides a pharmaceutical preparation for preventing or treating cystitis, said pharmaceutical preparation comprises a vitamin D compound and pharmaceutically acceptable carriers. The present invention provides a pharmaceutical preparation for preventing or treating urethritis, wherein the pharmaceutical preparation comprises a vitamin D compound and pharmaceutically acceptable carriers. The present invention provides a pharmaceutical preparation for preventing or treating urinary tract infections, wherein the pharmaceutical preparation comprises a vitamin D compound and pharmaceutically acceptable carriers. In a preferred embodiment, the vitamin D compound is a vitamin D receptor ligand (VDR ligand). In a more preferred embodiment, the vitamin D compound is a vitamin D receptor agonist. Preferably, the vitamin D compound is selected from vitamin D₂, vitamin D₃, as well as an isomer, activated form or analog thereof. Preferably, the vitamin D compound is selected from vitamin D₂, D₃ and its analogs. In certain embodiments, the vitamin D compound is an activated form of vitamin D, e.g., calcifediol (25-OH D₃) or calcitriol (1,25- (OH)₂ D₃). In certain preferred embodiments, the pharmaceutically acceptable carriers are substances described below, such as sugars, starches, cellulose and derivatives thereof, gelatin, talc, excipients, oils, polyhydric alcohols, esters, buffers, alginic acid or other non-toxic compatible substances used in pharmaceutical preparations. According to the dosage forms to be prepared, a variety of conventional excipients, such as disintegrants, lubricants, binders, antioxidants, complexing agents and other pharmaceutically acceptable carriers may be added to the composition of the invention. Any commonly used oral dosage forms, such as tablet, granule, capsule, oral liquid, and other forms, may be prepared by conventional formulation methods. The pharmaceutical preparation of the present invention may be in the form of a tablet, a capsule, an injection, an infusion, a suppository, an inhalant or an ointment, but is not limited thereto.

In the other aspect, the present invention relates to a kit for preventing or treating cystitis, urethritis and/or urinary tract infection. The kit comprises the pharmaceutical composition or pharmaceutical preparation as described above and instructions on how to use the compositions or pharmaceutical preparations. The present invention relates to a kit for preventing or treating cystitis. The kit comprises the pharmaceutical composition or pharmaceutical preparation as described above as well as instructions on how to use the composition or pharmaceutical formulation. The present invention relates to providing a kit for preventing or treating urethritis, and the kit comprises the pharmaceutical composition or pharmaceutical preparation as described above as well as instructions on how to use the composition or pharmaceutical preparation. The present invention relates to providing a kit for preventing or treating urinary tract infection, and the kit comprises the pharmaceutical composition or pharmaceutical preparation as described above as well as instructions on how to use the composition or pharmaceutical preparation.

### B. Definitions

Unless defined otherwise, all technical and scientific terms used herein have the same meaning as is commonly understood by one of ordinary personnel skilled in the art to which this invention belongs. All patents, applications, published applications and other publications referred to herein are incorporated by reference in their entirety. If a definition set forth in this section is contrary to or otherwise inconsistent with a definition set forth in the patents, applications, published applications and other publications that are herein incorporated by reference, the definition set forth in this section prevails over the definition that is incorporated herein by reference.

As used herein, the term "vitamin D compound" includes any vitamin D compounds used for preventing or treating cystitis, urethritis and/or urinary tract infections. In general, vitamin D compounds, being as vitamin D receptor ligands (VDR ligands) and being used for prevention or treatment of cystitis, urethritis and/or urinary tract infections, are considered to be within the scope of the present invention. The vitamin D compounds are preferably vitamin D receptor agonists. Thus, vitamin D compounds comprise ring-opening steroid, the example of specific vitamin D compounds applicable to the method of the present invention is described further herein. The Vitamin D compound include vitamin D₂ compound, vitamin D₃ compound, as well as an isomer, activated form or analog thereof. Preferred vitamin D compounds are vitamin D₃ compounds which are vitamin D receptor ligands (more preferably agonists). Vitamin D₂ and vitamin D₃ compounds are compounds including vitamin D₂, D₃ and their analogs. In certain embodiments, the vitamin D compounds may be steroids, such as ring opening steroids, e.g. calciferol (VD₃), calcifediol (25-OH D₃) or calcitriol (1,25 ( OH) ₂D₃)

The term "a second drug for prevention or treatment of cystitis, urethritis and/or urinary tract infection " refers to known drugs used for prevention or treatment of cystitis, urethritis and/or urinary tract infection, includes various existing antibiotics such as TMP-SMX, nitrofurantoin (nitrofurantoin), ciprofloxacin (ciprofloxacin), levofloxacin (levofloxacin) or chemical material, penicillin (penicillin), amoxicillin.

The term "pharmaceutically acceptable" means to be suitable for use in contact with human and animal tissue, without excessive toxicity, irritation, allergic response, or other problem or complication within the scope of sound medical judgment.

The term "pharmaceutically acceptable carriers" comprises pharmaceutically acceptable materials, compositions or vehicles, such as liquid or solid filler, diluent, excipient, solvent or encapsulating material, which participate in carrying or transporting the chemical from one organ, or a body part in the body to another organ or another part. Each carrier must be "acceptable" in the sense of being compatible with the other ingredients of the formulation and harmless to the patient.

The term "administration" includes the introduction to the subject of the vitamin D compound to exert its intended function. The routes of administration comprise injection (subcutaneous, intravenous, parenteral, intraperitoneal), oral administration, inhalation, rectal, transdermal, or via bladder instillation. Oral administration is preferred. Preparations of the present invention can be administered orally or via intravenous, subcutaneous, intramuscular, transdermal, nasal, or rectal route of administration, or by inhalation. Dosage forms for oral administration include tablet, pill, powder, granule, liquid, suspension, syrup, capsule and the like.

### BRIEF DESCRIPTION OF THE DRAWINGS

Fig. 1 shows an antibacterial experiment about VD₃ stimulating the bladder epithelium. Each well of six-well plates was added 10 µL of the test solution at different concentrations or vehicle.
Fig. 2 shows the colony-forming unit (CFU) of 5637 cell lysate of each dose group in an antibacterial experiment about VD₃ stimulating the bladder epithelium.
Fig. 3 shows the statistical results of repeated experiments, wherein the CFU in each dose group of 5637 cells lysate was counted and the CFU ratio of each group and ethanol solvent control group was calculated to get a relative CFU in an antibacterial experiments about VD₃ stimulating the bladder epithelium. The method of statistical test is the One-Way ANOVA LSD Test. * indicates t <0.05 compared with the control group of ethanol, ** represents t <0.01compared with the control group of ethanol.
Fig. 4 shows the ELISA test result of LL-37 in each dose group of lysate in an antibacterial experiment about VD₃ stimulating the bladder epithelium.
Fig. 5 shows the CFU of SV-HUC1 cell lysate of each dose group in an antibacterial experiment about VD3 stimulating the bladder epithelium.

### EXAMPLES

### Example 1: Antibacterial experiment about VD₃ stimulating lymphocytes

### 1. Experimental objective

In this study, the final activated form of vitamin D, 1,25-dihydroxyvitamin D₃ [1,25 (OH)₂D₃] was the test substance. The lymphoma U-937 cells of human tissue cell were used as experimental systems, the E.coli O111B4 was the antibacterial target. Cell supernatants treated by 1,25(OH)₂D₃ was co-incubated with E. coli 0111B4. The E. coli 0111B4 colony forming units (CFU) was used to examine whether 1,25(OH)₂D₃ achieved an antibacterial effect by stimulating cell lines derived from human to secrete antimicrobial peptides in vitro.

### 2. Experimental material

### 2.2 The information of the test substance 1,25(OH)₂D₃

| Name of drug | Supplier | Art. No. | Batch Number | preservation condition |
|---|---|---|---|---|
| 1,25 (OH)₂D3 | Sigma | D1530 | 023M4010V | -20 |

1,25(OH)₂D₃ was dissolved in anhydrous ethanol solution to formulate a stock solution at a concentration of 200 x 10⁻⁶M. After subpackage, the solution was stored in a refrigerator at - 20 until needed.

### 2.3 Experimental apparatus

MODEL 3111 CO₂ incubator
XDS-1B inverted microscope
SDG-D2 clean bench
DK-6000 water bath kettle
Biofuge Stratos centrifugal machine
SMARTSPEC 3000 ultraviolet spectrophotometer
SCS-24 thermostatic shakers

### 2.3 Bacterial and cell culture media

RPMI 1640 basal medium: Each 10.4g freeze-dried powder was supplemented with 1.5g sodium bicarbonate, 2.5g glucose and 0.11g sodium pyruvate. Firstly the mixture was dissolved in 800 ml of deionized water until completely dissolved. The solution was adjusted to pH 7.2-7.4 with 1 N HCl and was diluted to 1L with deionized water. Then the solution was filtered by a membrane filter with pore size of 0.22 µm and then subpackaged in blue cap bottles of 500 mL, stored in a refrigerator at 2-8 until needed.

RPMI 1640 complete media containing 10% FBS: Each 450 mL basal medium was supplemented with 50mL fetal bovine serum (FBS).

RPMI 1640 medium incomplete media containing 1% FBS: Each 49 mL basal medium was supplemented with 1 mL fetal bovine serum (FBS).

LB liquid medium: 10 g tryptone, 5 g yeast extract, 10 g sodium chloride were weighed accurately, and were dissolved in 900 mL deionized water. The pH of the solution was adjusted to 7.0 by 5N NaOH. The solution was diluted to 1 L by deionized water, and was autoclaved at 121 for 15 min.

1.5% agar plates: each 1000 mL LB broth was supplemented with 15 g agar powder, and was autoclaved at 121 for 15 min. 2 mL LB medium containing 1.5% agar was added to each well of the 6 well plate. After the medium solidified, the 1.5% agar plates was put into a refrigerator at 2-8 .

Top agar medium: each 100 mL LB broth was supplemented with 0.8g agar powder to prepare top agar medium containing 0.8% agar, and was autoclaved at 121 for 15 min. After the medium cooling, the top medium was placed in the 2-8 refrigerator.

### 3. Experimental method

### 3.1 Cell inoculation and administration (Day 1)

U-937 cells in the condition of exponential growth were centrifuged (1000 rpm, 5 min); After centrifugation, the supernatant was discarded, and cell sediment was resuspended in an appropriate amount of RPMI1640 incomplete medium to make sure cells dispersed uniformly;

The cell suspension was counted by hemacytometer counting ;

The concentration of cell suspension was adjusted to 1 x 10⁵/mL using RPMI 1640 incomplete medium;

Each well of 6-well plate was inoculated with 3mL cell suspension, the total number of cells per well was 3 x 10⁵;

According to the label and the experimental design, 10 µL 200x test solution or vehicle of various concentrations with 0.5% (v/v) dose volume was added to each well.

Each dose was repeated in two parallel wells, one well harvested after 24 hours, and the other well harvested after 48 hours;

After administration, the 6-well plate was placed in 37 , 5% CO₂ incubator, cultured for 24 hours and 48 hours respectively.

### 3.2 Preparation of the E. coli infection bacteria solution

Day 1 afternoon, a E. coli O111B4 glycerol pipe was thawed, 10 µL glycerol bacteria was inoculated in 10 mL of LB liquid medium without any antibiotic, and was cultured on oscillator at 37 overnight;

Day 2 morning, 200 µL cultured broth (1:100 ratio) cultured overnight was transferred to 20 mL LB liquid medium without any antibiotics and was cultured on oscillator at 37 until OD600 was 0.6-1.0, then the next step was antibacterial experiment (Day 3); the remaining overnight cultured broth was stored in the refrigerator at 2-8 until needed.

Day 2 afternoon, 10 µL overnight cultured broth was inoculated in 10 mL LB medium without any antibiotics, and was cultured on oscillator at 37 overninght;

Day 3 morning, 200 µL overnight cultured broth (1: 100 ratio) was transferred to 20 mL LB medium without any antibiotics, and was cultured on oscillator at 37 until OD600 was 0.6-1.0, then the next step was antibacterial experiment (Day 3).

### 3.3 Antimicrobial Test (Day 2 and Day 3)

Before the experiment started, 1.5% agar plate (lower medium) prepared beforehand was placed at room temperature for at least one hour;
Top agar medium (containing 0.8% agar) was melted, and packaged. Each 1.6 mL of Top agar medium was packaged in each tube, and then the small tube was placed in 45 water bath to keep warm;
10 mL E. coli bacteria of OD600 0.6-1.0 was transferred into 50mL centrifuge tubes to centrifuge (1000 rpm x 5min); After centrifugation, the supernatant was discarded, the bacteria precipitate was resuspended by adding 10 mL PBS; the above-described bacterial suspension was diluted 1x10⁵ fold in PBS dilution by multiple proportion dilution (from 10-fold);
After the drug action finished (24 hours or 48 hours), U-937 cells was centrifugated (1000 rpm, 5 min). After centrifugation, the supernatant was drawn back;
Each centrifuge tube previously added 50 µL diluted bacterial suspension (approximately 150-250 bacteria);
Bacterial suspension were added with 1.2 mL cell supernatant from each cell supernatant, and mixed;
The supernatant / bacterial mixture was placed on shaking table (200 rpm) at 37 and was incubated for 90 minutes. After incubation, the next step was plating;
Plating: 0.8 mL of cell supernatant/broth mixture was mixed with 1.6 mL top agar medium, the mixture was plated on 1.5% agar plates, 600 uL per well in triplicate.

Sterility test: 800 uL PBS and 1.6 mL top agar medium was mixed, and plated as above. Antibiotics control experiment: 700 uL PBS, 50 uL broth, 2 uL antibiotic ampicillin, 1.6 mL top agar were mixed, and plated as above.

After top agar medium was cooled and solidified, all 6-well plates (agar plates)were put into 37 incubator and cultured overnight.

### 3.4 Colony count (Day3 and Day4)

The next day after plating, the 6-well plates were took out from the incubator for colony counting.

### 4. Results

U937 cells were induced by vitamin D3 for 24h and 48h, the supernatant was used for antibacterial experiments. The colony forming units (CFU) of each dose group were calculated. The results were as follows:

| | Concentration of VD3 | Day | Serum | CFU |
|---|---|---|---|---|
| High dose | 10⁻⁷M | 1 | 10% | 172 |
| | 10⁻⁷M | 2 | 10% | 127 |
| | 10⁻⁷M | 1 | 1% | 108 |
| | 10⁻⁷M | 2 | 1% | 112 |
| Low Dose | 10⁻⁸M | 1 | 10% | 112 |
| | 10⁻⁸M | 2 | 10% | 97 |
| | 10⁻⁸M | 1 | 1% | 102 |
| | 10⁻⁸M | 2 | 1% | 93 |
| Control | 0 | 2 | 10% | 271 |
| | 0 | 2 | 1% | 241 |

According to the result, the sterile test group and antibiotic control group had no colonies produced. From the available data, after stimulating U937 cells by different concentrations of VD₃, the supernatant solution showed significant antibacterial effect.

### 5. Discussion

In order to investigate the role of vitamin D₃ in prevention and treatment of cystitis, urethritis, and/or urinary tract infection in the present study, lymphatic U937 cells derived from human was stimulated by vitamin D₃, and supernatant was collected for antibacterial experiments. The antibacterial effect of cell supernatant was investigated.

The experimental system was stable, and the experimental results were controlled within a reasonable margin of error, so the data was reliable. The results showed that after stimulating U937 cells by VD3, the supernatant solution showed significant antibacterial effect.

### Example 2: Antibacterial experiment about VD₃ stimulating epithelial cells

### 1. Experimental objective

In this study, the final activated form of vitamin D, 1,25-dihydroxyvitamin D3 [1,25(OH)2D3] was the test substance, the human bladder cancer cells 5637 was used as experimental systems, the E.coli CFT073 was antibacterial target, cells treated by 1,25(OH)2D3 was co-incubated with E.coli CFT073. The E. coli CFT073 colony forming units (CFU) was used to examine whether 1,25(OH)₂D₃ achieved an antibacterial effect by stimulating cell lines derived from human to secrete antimicrobial peptides in vitro.

### 2. Experimental material

### 2.1 The information of the test substance 1, 25(OH)₂D₃

| Name of drug | Supplier | Art. No. | Batch Number | preservation condition |
|---|---|---|---|---|
| 1,25(OH)₂D₃ | Sigma | D1530 | 023M4010V | -20 |

1,25(OH)₂D₃ was dissolved in anhydrous ethanol to form a stock solution at a concentration of 200 x 10⁻⁶ M. After subpackaged, the solution was stored in a refrigerator at -20 until needed.

### 2.2 Experimental apparatus

MODEL 3111 CO2 incubator
XDS-1B inverted microscope
SDG-D2 clean bench
DK-6000 water bath kettle
Biofuge Stratos centrifugal machine
SMARTSPEC 3000 ultraviolet spectrophotometer
SCS-24 thermostatic shakers

### 2.3 Bacterial and cell culture media

RPMI 1640 basal medium: each 10.4g freeze-dried powder was supplemented with 1.5g sodium bicarbonate, 2.5g glucose and 0.11g sodium pyruvate. The mixiture were firstly dissolved in 800 ml of deionized water. After completely dissolved, the solution was adjusted to pH 7.2-7.4 by 1 N HCl and diluted to 1L by deionized water. Then the basal medium was filtered to sterilize by a membrane filter with the pore size of 0.22 µm and then subpackaged in blue cap bottles of 500 mL, stored in a refrigerator at 2-8 until needed.

RPMI 1640 complete media containing 10% FBS: each 450 mL basal medium was added 50mL fetal bovine serum (FBS).

### RPMI 1640 incomplete media without FBS.

5XLBE liquid medium: namely LB medium added Medium E. Tryptone 5 g, yeast extract 2.5 g, sodium chloride 5g, MgSO₄ • 7H₂O 0.0986g, Citric Acid • H₂O 1.0087g, K₂HPO₄ 6.55g, and NaNH₄HPO₄ • 4H₂O 1.6726g were weighed accurately and dissolved in 90mL deionized water. The pH was adjusted to 7.0 by 5N NaOH, and was diluted with deionized water to 100mL, then sterilized by autoclave at 121 for 15 min.

0.8% agar plates: each 1000 mL LB liquid medium was added 8 g agar powder, and was autoclaved at 121 for 15 min. Each well of 6 well plate was added 1 mL LB medium containing 0.8% agar, until the medium solidified, 0.8% agar plates wad stored in a refrigerator at 2-8 (or prepared freshly on the day of experiment).

Top 0.8% agar medium: Each 100 mL LB broth was added 0.8g agar powder to prepare top agar medium containing 0.8% agar, sterilized by autoclave at 121 for 15 min. When the medium cooled, the top medium was placed in a refrigerator at 2-8.

### 3. Experimental method

### 3.1 Antibacterial experiment

### 3.1.1 Cell inoculation (Day 1)

5637 cells in the condition of exponential growth and about 90% confluence were digested by pancreatin (GIBCO, Cat. No. 25200-056) for 5min. When most of the cells came off from the culture plate, complete medium was added to terminate digestion. The non-peeling cells were blown down from the culture plate by a pipette, and were transferred to a 15ml centrifuge tube. The cells were centrifuged at 1500 rpm for three minutes. The supernatant was discarded. The cell deposit was resuspended in complete medium to ensure that the cells uniformly dispersed.

The cell suspension was counted by hemacytometer;

The concentration of cell suspension was adjusted to 4 x 10⁵/mL using incomplete RPMI 1640 medium;

Each well of 6-well plate was inoculated with 2mL cell suspension, the total number of cells per well was 8 x 10⁵;

6-well plate was placed in an incubator under the condition of 37, 5% CO₂.

### 3.1.2 Administration (Day 2)

2ml complete medium was replaced with 2ml incomplete medium in each well. According to the label and experimental design, each well was added 10 µL of the test solution at different concentrations or vehicle, with 0.5% (v/v) dose volume.

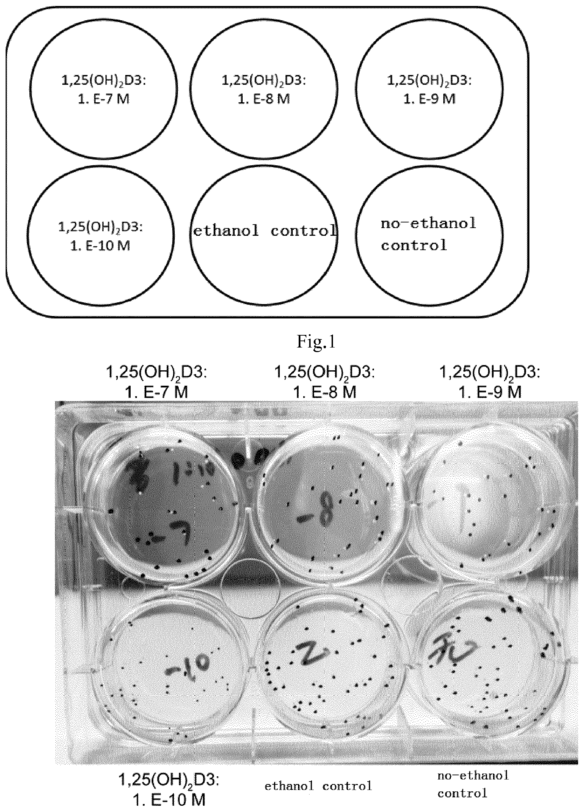

The wells treated were showed in Fig 1.

### 3.1.3 Preparation of the E. coli infection bacteria solution (Day 2&3)

Day 2 afternoon, a E. coli CFT073 glycerol tube was thawed, 10 µL glycerol bacteria was inoculated in 10 mL of LB liquid medium without any antibiotic, shaken at 37 overnight; Day 3 morning, 50 µL overnight cultured broth (1:100 ratio) was transferred to 5 mL LB liquid medium without any antibiotics, shaken to culture at 37 until OD600 as 0.9-1.0, so the next step antibacterial experiment could be proceeded.

### 3.1.4 Antimicrobial Test (Day 3)

Before the experiment started, agar plate containing 0.8% (lower medium) prepared beforehand was placed at room temperature (or prepared freshly);

Top agar medium (containing 0.8% agar) was melted, and placed in water bath at 50.

An appropriate amount of E. coli bacteria with OD600 0.9 to 1.0 was diluted to OD600 of 0.83 (equivalent to 5X10⁸ CFU/mL); 24 hours later, bacterial suspension diluted by 10µL PBS was added to each well (approximately 3.125X10⁵ bacteria);

Cells added bacteria were cultured in an incubator for 2 hours at 37.

After incubation, the supernatant was drawn to sterile EP tube. Cells were washed three times with sterile PBS to remove bacteria not adhered on the cell surface. Cells were lysed by 500µL PBS solution containing 1% Triton X-100 and protease inhibitors.

Plating: cell lysate was serially diluted with PBS by 10,000 fold. 100µL diluted cell lysate was put into 1.5 mL EP tube, added 900µL 0.8% LB medium, and dissociated uniformly.

Then the cell lysate was added to 6-well plates paved by a layer of LB medium. Make ensure to plate uniformly. After medium solidification, a layer of 1mL/cell LB medium was paved upon the medium. Make ensure to plate uniformly.

When the top agar medium was cool and solidified, all 6-well plates (agar plates) were cultured in an incubator at 37 overnight.

### 3.1.5 Colony count (Day4)

The next day, the 6-well plates were took out from the incubator for colony counting.

### 3.2 ELISA experiments about effect of 1,25 (OH)₂D₃ on LL-37 production

### 3.2.1 The procedure of inoculation (Day1), administration (Day2), and E. coli infection bacteria solution preparation (Day 2&3) was the same as 3.1.1, 3.1.2 and 3.1.3.

### 3.2.2 ELISA test of LL37

Cell lysate was obtained using the method of 3.1.4. Cell debris and bacteria were removed by 13000 rpm centrifugation for 5min. Lysate supernatant was fetched out and LL-37 was quantified by Human LL-37 ELISA Kit (Hycult, Cat. No. HK321).

### 4. Results

### 4.1 Antibacterial experiment

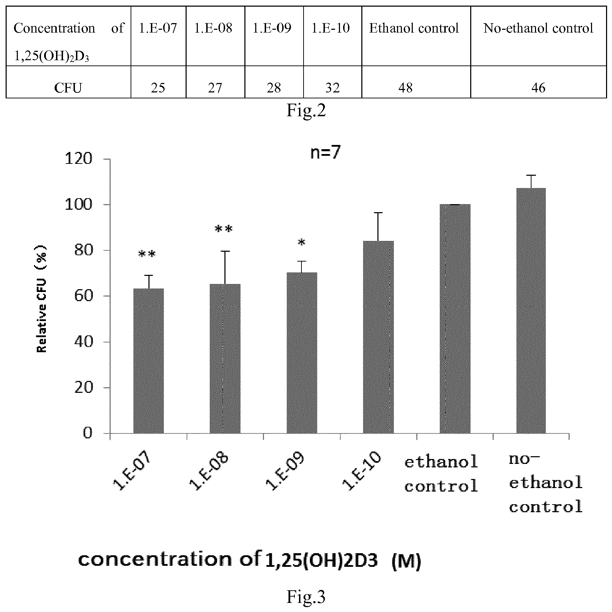

Colony forming units (CFU) of 5637 cell lysates in each dose group were marked by a marker pen. The representative results were shown in Fig 2.

CFU of 5637cells lysate in each dose group were counted, and the ratio of CFU in each group to ethanol solvent control group was calculated to get relative CFU. Statistical results of the repeated experiments were shown in Fig. 3.

Statistical test was the One-Way ANOVA LSD Test. * indicated t <0.05 compared with control group of ethanol, ** indicated t <0.01 compared with control group of ethanol.

From the available data, 5637 cells stimulated by VD3 at different concentrations showed significant antibacterial effect.

### 4.2 ELISA experiments about effect of 1,25 (OH)₂D₃ on LL-37 production

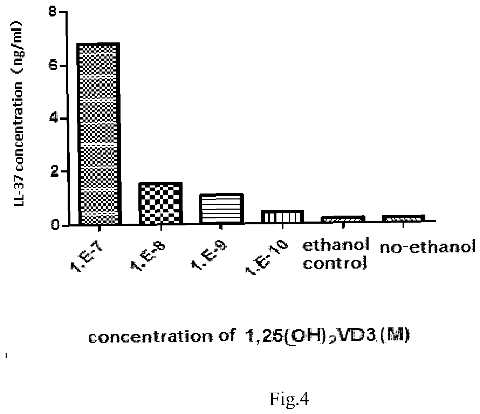

The results of LL-37 ELISA test for each dose group lysate were shown in Fig. 4.

From the available data, the stimulation of 5637 cells with VD3 at different concentrations leaded to significant dose-dependent increase of LL-37 levels in cell lysates.

### 5. Discussion

In order to investigate the role of vitamin D₃ in prevention and treatment of cystitis, in this study, 5637 cell derived from human bladder cells were stimulated by vitamin D₃ and co-incubated with E. coli to investigate the antibacterial effect of cell lysate.

The experimental system was stable, and the experimental result was controlled within a reasonable margin of error, so the data was reliable. The results show that the cell lysates have significant antibacterial activity after 5637 cells stimulated by VD₃ at different concentrations.

### Example 3: Antibacterial experiment about VD3 stimulating urethral epithelial cells

### 1. Experimental objective

In this study, with the final activated form of vitamin D₃, 1,25-dihydroxyvitamin D₃ [1,25 (OH)₂D₃] as the test substance, the immortalized human urethral epithelial cells SV- HUC1 as experimental systems, the E.coli CFT073 as antibacterial target, the cells treated by 1,25 (OH) 2D3 were co-incubated with E.coli CFT073. The E. coli CFT073 colony forming units (CFU) were used to examine whether 1,25 (OH) 2D3 achieves an antibacterial effect by stimulating cell lines derived from human to secrete antimicrobial peptides in vitro.

### 2. Experimental material

### 2.1 The information of the test substance 1, 25 (OH)₂D₃

| Name of drug | Supplier | Art. No. | Batch Number | preservation condition |
|---|---|---|---|---|
| 1,25 (OH)₂D₃ | Sigma | D1530 | 023M4010V | -20 |

| | | | | |
|---|---|---|---|---|
| 1,25 (OH)₂D₃ was dissolved in anhydrous ethanol to form a stock solution at a concentration of 200 x 10⁻⁶ M. After subpackage, the solution was stored in the refrigerator at -20 until needed. | | | | |

### 2.2 Experimental apparatus

MODEL 3111 CO2 incubator
XDS-1B inverted microscope
SDG-D2 clean bench
DK-6000 water bath kettle
Biofuge Stratos centrifugal machine
SMARTSPEC 3000 ultraviolet spectrophotometer
SCS-24 thermostatic shakers

### 2.3 Bacterial and cell culture media

F12 basal medium: purchased from Hyclone, Product code SH30026.01B, 500 mL/bottle, stored in a refrigerator at 2-8 until needed.

F12 complete media containing 10% FBS: each 450 mL basal medium was added 50mL fetal bovine serum (FBS).

F12 basal medium without FBS, namely incomplete media.

5XLBE liquid medium: namely LB medium added Medium E. Tryptone 5 g, yeast extract 2.5 g, sodium chloride 5g, MgSO₄ • 7H20 0.0986g, Citric Acid • H2O 1.0087g, K₂HPO₄ 6.55g, NaNH₄HPO₄ • 4H₂O 1.6726g were weighed accurately and dissolved in 90mL deionized water. The pH was adjusted to 7.0 by 5N NaOH. The solution was diluted to 100mL with deionized water, then sterilized by autoclave at 121 for 15 min.

0.8% agar plates: each 1000 mL LB liquid medium was added 8 g agar powder, and was autoclaved at 121 for 15 min. Each well of 6 well plate was added 1 mL LB medium containing 0.8% agar. After the medium solidified, 0.8% agar plates was stored in a refrigerator at 2-8 (or prepared freshly on the day of experiment).

0.8% top agar medium: Each 100 mL LB broth was added 0.8g agar powder to prepare top agar medium containing 0.8% agar, then sterilized by autoclave at 121 for 15 min. After the medium cooling, the top medium was placed in the refrigerator at 2-8.

### 3. Experimental method

### 3.1 Antibacterial experiment

### 3.1.1 Cell inoculation (Day 1)

SV-HUC1 cells in the condition of exponential growth and about 90% confluence were digested by pancreatin (GIBCO, Cat. No. 25200-056) for 5min. When most of the cells came off from the culture plate, complete medium was added to terminate digestion. The non-peeling cells were blown down from the culture plate by a pipette, and were transferred to a 15ml centrifuge tube. The cells were centrifuged at 1500 rpm for three minutes. The supernatant was discarded. The cell deposit was resuspended in complete medium to ensure that the cells dispersed uniformly.

The cell suspension was counted by hemacytometer;

The concentration of cell suspension was adjusted to 4x10⁵/mL using F12 incomplete medium;

Each well of 6-well plate was inoculated with 2mL cell suspension, the total number of cells per well was 8 x 10⁵;

6-well plate was placed in the incubator under the condition of 37, 5% CO₂.

### 3.1.2 Administration (Day 2)

2ml complete medium was replaced with 2ml incomplete medium in each well. According to the label and experimental design, each well was added 10 µL of the test solution at different concentrations or vehicle, with 0.5% (v/v) dose volume.

The wells were treated as shown in Fig. 1.

### 3.1.3 Preparation of the E. coli infection bacteria solution (Day 2&3)

Day 2 afternoon, a E. coli CFT073 glycerol tube was unfrozen, 10 µL glycerol bacteria was inoculated in 10 mL LB liquid medium without any antibiotic, and was shaken at 37 overnight;

Day 3 morning, 50 µL overnight culture broth (1:100 ratio) was transferred to 5 mL LB liquid medium without any antibiotics, and was shaken at 37 until OD600 was 0.9-1.0, so the next step antibacterial experiment could be proceeded.

### 3.1.4 Antimicrobial Test (Day 3)

Before the experiment started, agar plate containing 0.8% (lower medium) prepared beforehand was placed at room temperature (or prepared freshly);

Top agar medium (containing 0.8% agar) was melted, and placed in water bath at 50.

An appropriate amount of E. coli bacteria with OD600 of 0.9 to 1.0 was diluted to OD600 of 0.83 (equivalent to 5X10⁸ CFU/mL); 24 hours later, bacterial suspension diluted by 10µL PBS was added to each well (approximately 3.125 X 10⁵ bacteria);

Cells added bacterial were cultured in an incubator for 2 hours at 37.

After incubation, the supernatant was drawn to sterile EP tube. Cells were washed three times with sterile PBS to remove bacteria not adhered on the cell surface. Cells were lysed by 500µL PBS solution containing 1% Triton X-100 and protease inhibitors.

Plating: cell lysate and supernatant were serially diluted with PBS by 10,000 fold and 100000 fold separately. 100µL diluted cell lysate was put into 1.5mL EP tube, added 900µL 0.8% LB medium, and dissociated uniformly. Then the cell lysate was added to 6-well plates paved by a layer of LB medium. Make ensure to plate uniformly. After medium solidification, a layer of LB medium was paved upon the medium. Make ensure to plate uniformly.

When the top agar medium was cooled and solidified, all 6-well plates (agar) were put in incubator at 37 overnight.

### 3.1.5 Colony count (Day4)

The next day, the 6-well plates were took out from the incubator for colony counting.

### 4. Results

### 4.1 Antibacterial experiment

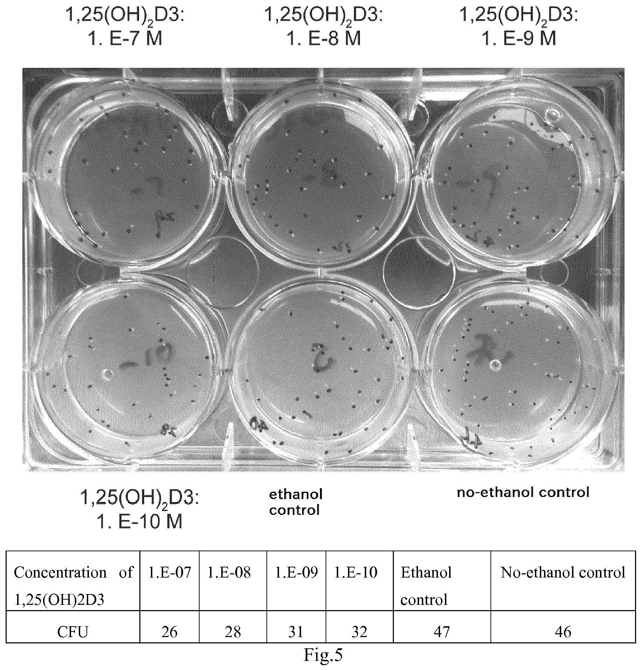

Colony forming units (CFU) of SV-HUC1 cell lysate in each dose group was marked by a marker pen. The representative results were shown in Fig. 5.

### 5. Discussion

In order to investigate the role of vitamin D₃ in prevention and treatment of urethritis, in this study, SV-HUC1 cells derived from human were stimulated by vitamin D₃ and co-incubated with E. coli to investigate the antibacterial effect of VD₃.

The experimental system was stable, and the experimental result was controlled within a reasonable margin of error, so the data was reliable. The results show that the cell lysates have significant antibacterial activity after SV-HUC1 cells stimulated by VD₃ at different concentrations.

In order to describe and understand the present invention more clearly, we describe the present invention by examples in detail. It is clear that modification and alterations of the present invention will be apparent to those skilled in the art without departing from the scope and spirit of the present invention.

## Claims

1. Use of a vitamin D compound in the preparation of a medicament for preventing or treating cystitis.

2. The use of claim 1, wherein the vitamin D compound is selected from vitamin D₂, vitamin D₃, and an activated form or analog of vitamin D₃.

3. The use of claim 2, wherein the activated form of vitamin D₃ is 25-hydroxyvitamin D₃ or 1,25-dihydroxyvitamin D₃.

4. The use of claim 2, wherein the vitamin D compound is 1,25-dihydroxyvitamin D₃.

5. A pharmaceutical composition for prevention or treatment of cystitis, **characterized in** comprising a vitamin D compound and a second drug used for prevention or treatment of cystitis.

6. The pharmaceutical composition of claim 5, wherein said vitamin D compound is selected from vitamin D₂, vitamin D₃, and an activated form or analog of vitamin D₃.

7. A pharmaceutical preparation for prevention or treatment of cystitis, **characterized in** comprising a vitamin D compound and pharmaceutically acceptable carriers.

8. The pharmaceutical preparation of claim 7, wherein the vitamin D compound is selected from vitamin D₂, vitamin D₃, and an activated form or analog of vitamin D₃.

9. The pharmaceutical preparation of claim 7, wherein said pharmaceutical preparation is in the form of a tablet, a capsule, an injection, an infusion, a suppository, an inhalant or an ointment.

10. A kit for prevention or treatment of cystitis, **characterized in** comprising the pharmaceutical preparation of claim 7 and instructions on how to use the pharmaceutical preparation.

11. Use of a vitamin D compound in preparation of a medicament for prevention or treatment of urethritis.

12. The use of claim 11, wherein the vitamin D compound is selected from vitamin D₂, vitamin D₃, and an activated form or analog of vitamin D₃.

13. The use of claim 12, wherein the activated form of vitamin D₃ is 25-hydroxyvitamin D₃ or 1,25-dihydroxyvitamin D₃.

14. The use of claim 12, wherein the vitamin D compound is 1,25-dihydroxyvitamin D₃.

15. A pharmaceutical composition for prevention or treatment of urethritis, **characterized in** comprising a vitamin D compound and a second drug used for prevention or treatment of urethritis.

16. The pharmaceutical composition of claim 15, wherein said vitamin D compound is selected from vitamin D₂, vitamin D₃, and an activated form or analog of vitamin D₃.

17. A pharmaceutical preparation for prevention or treatment of urethritis, **characterized in** comprising a vitamin D compound and pharmaceutically acceptable carriers.

18. The pharmaceutical preparation of claim 17, wherein the vitamin D compound is selected from vitamin D₂, vitamin D₃, and an activated form or analog of vitamin D₃.

19. A pharmaceutical preparation of claim 17, which the pharmaceutical preparation is in the form of a tablet, a capsule, an injection, an infusion, a suppository, an inhalant or an ointment.

20. A kit for prevention or treatment of urethritis, **characterized in** comprising the pharmaceutical preparation of claim 17 and instructions on how to use the pharmaceutical preparation.
